# EUROPEAN PATENT SPECIFICATION

(11) **EP 3 244 899 B1**
(45) Date of publication and mention of the grant of the patent: **24.11.2021**
(21) Application number: 16737166.5
(22) Date of filing: 13.01.2016
(51) Int. Cl.: A61K 31/55, A61P 15/00, A61K 31/39, A61K 31/26

(54) **PROSTAGLANDIN TRANSPORTER INHIBITORS FOR INHIBITING OVULATION**
PROSTAGLANDINTRANSPORTER-INHIBITOREN ZUR HEMMUNG DES EISPRUNGS
INHIBITEURS DE TRANSPORTEUR DE PROSTAGLANDINES POUR INHIBER L'OVULATION

(30) Priority: 14.01.2015 US 201562103091 P
(43) Date of publication of application: 22.11.2017
(73) Proprietor: Tel Hashomer Medical Research Infrastructure and Services Ltd., Tel Hashomer, 52621 Ramat-Gan (IL)
(72) Inventor: HOURVITZ, Ariel, 5296000 Ramat Gan (IL); YUNG, Yuval, 79505 Nir Israel (IL); MARKMAN, Svetlana, 7740223 Ashdod (IL); YERUSHALMI, Gil, 4723119 Ramat Ha'Sharon (IL)
(74) Representative: Gassner, Birgitta
(86) International application number: PCT/IL2016/050036
(87) International publication number: WO 2016/113734

(56) References cited:
- WO-A2-2007/136638
- US-A1- 2009 233 933
- J.E. FORTUNE ET AL. THE PERIOVULATORY PERIOD IN CATTLE: PROGESTERONE, PROSTAGLANDINS, OXYTOCIN AND ADAMTS PROTEASES vol. 6, no. 1, 12 April 2010, ITHACA, NY , 14853, USA, pages 60 - 71, XP055464519
- MILO C WILTBANK ET AL.: 'Regulation of intraluteal production of prostaglandins' REPRODUCTIVE BIOLOGY AND ENDOCRINOLOGY 10 November 2003, page 3, XP021009364
- K.M. KLOHONATZ ET AL.: 'The effects of a prostaglandin transport inhibitor (DIDS) on delaying diestrus in non-pregnant mares' JOURNAL OF EQUINE VETERINARY SCIENCE vol. 35, 01 May 2015, FORT COLLINS, CO, USA ;, pages 418 - 419, XP029158384
- Brenda S Chan ET AL: "Mechanism of Prostaglandin E 2 Transport across the Plasma Membrane of HeLa Cells and Xenopus Oocytes Expressing the Prostaglandin Transporter "PGT"", Journal of Biological Chemistry, vol. 273, no. 12, 1 March 1998 (1998-03-01), pages 6689-6697, XP055691069,
- CHI Y ET AL: "Identification of a New Class of Prostaglandin Transporter Inhibitors and Characterization of Their Biological Effects on Prostaglandin E2 Transport", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 316, no. 3, 1 March 2006 (2006-03-01) , pages 1346-1350, XP008102514, ISSN: 0022-3565, DOI: 10.1124/JPET.105.091975 [retrieved on 2005-11-03]

## Description

### FIELD OF THE INVENTION

The present invention provides methods for inhibiting ovulation in a female subject in need thereof, including a subject undergoing fertility treatments. The methods comprise administering to the female subject one or more prostaglandin transporter (PGT) inhibitors.

### BACKGROUND OF THE INVENTION

Prostaglandins (PGs) are a group of hormone-like lipid compounds that are derived enzymatically from fatty acids and have important physiological functions. PGs contain 20 carbon atoms, including a 5-carbon ring. PGs are synthesized from arachidonic acid by cyclooxygenases (COX1 and COX2) and corresponding synthases.

PGs are local mediators of a variety of biological and pathological processes, where their biological effects include triggering inflammation, fever and pain; inducing labor; modulating renal hemodynamics and of water and solute reabsorption; and causing constriction or dilation in vascular smooth muscle cells. Further, PGs have long been known to participate in female reproductive functions, including ovulation, fertilization, luteolysis, implantation and parturition. As such, PGs are known as one of the major intraovarian mediators of the ovulatory cascade.

At the cellular level, PGs are involved in several major signaling pathways, including the MAP kinase and protein kinase A pathways by upregulation of cAMP. At physiological pH, PGs predominate as charged anions and diffuse poorly through plasma membranes despite their lipid nature. Therefore, PGs require a carrier-mediated transport mechanism to cross biological membranes - the PG transporter (PGT).

It has been recently shown that intrauterine inhibition of PGT inhibits the pulsatile release of PGF2(alpha) from the endometrium without modulating spatial expressions of estrogen and oxytocin receptor proteins and metabolism of PGF2(alpha) at the time of luteolysis (Lee J. et al., Biol. Reprod. 2013,89(2):27).

Lee J. et al., showed that at the time of luteolysis, transport of PGF2(alpha) from uterus to ovary through the utero-ovarian plexus is regulated by PGT-mediated mechanisms (Lee J. et al., Endocrinology, 2010, 151(7):3326-3335).

Sakhila K. Banu et al. established that PGT has a role in the cellular transport of PGs in endometrium and myometrium, and in compartmental transport of endometrial PGs in utero-ovarian plexus (UOP) in the bovine reproductive system (Sakhila K. Banu et al., Proc. Natl. Acad. Sci. U S A,, 2003; 100(20): 11747-11752).

US patent No. 8,227,466 discloses PGT inhibitors.

In a recent study it was shown that PGT is expressed in human granulosa cells following induction by hCG and is among the highest differentially expressed transcripts showing 392 fold induction in expanded cumulus cells as compared to compact cumulus cells (CCs; Yerushalmi G.M. et al., Mol Hum Rep. 2014, Aug;20(8):719-735).

Fortune et al. (2009) Anim Reprod, 6(1): 60 -71 summarize studies on the temporal patterns, roles, regulation, and interrelationships among progesterone (P4) and its receptor (PGR), prostaglandins (PGs) and oxytocin (OT) in bovine periovulatory follicles.

There is an unmet need for safe and effective contraception platforms which are reversible, devoid of hormonal manipulations and which do not interfere with sexual intercourse.

### SUMMARY OF THE INVENTION

The invention is directed to methods, compositions and kits for inhibiting ovulation in a female subject. The methods of the invention comprise administering a prostaglandin transporter (PGT) inhibitor to female subjects in need of contraceptives. Advantageously, the PGT inhibitor is non hormonal and may be prescribed for administration only during a particularly short period within the menstrual cycle rather than daily.

To date, progesterone- and estrogen-based hormonal contraceptives administered, for example, in the form of pills and patches, are vastly used by women. Even though those contraceptives are effective as birth control, concern remains regarding their sideeffects and long-term use effect because of the widespread action of these hormones in many tissues. Moreover, some women are prohibited from taking hormonal contraceptives due to increased risk in developing serious medical illnesses such as heart attack, migraines and blood clots. Thus, providing non-hormonal effective and safe contraceptives for women is important for public health.

It is now disclosed for the first time that ovulation may be inhibited by PGT inhibitors. As exemplified herein, inhibition of PGT by any of the inhibitors 4,4'-Diisothiocyanatostilbene-2,2'-disulfonic acid (DIDS) and Bromocresol Green (BCG) significantly blocks ovulation in mice undergoing hormonal stimulation.

Thus, the present invention provides non-hormonal contraceptives which particularly block PGT and thereby inhibit ovulation. The present invention is based, in part, on the unexpected discovery that inhibition of PGT abolishes ovulation. Moreover, use of PGT inhibitors, such as, 4,4'-Diisothiocyanatostilbene-2,2'-disulfonic acid (DIDS) and Bromocresol Green (BCG) is effective when administered along with human chorionic gonadotropin (hCG). The hormone human chorionic gonadotropin (hCG) is analogue to luteinizing hormone (LH), and induces ovulation. In view of the above, the PGT inhibitors of the invention are useful when administered in proximity to LH surge.

According to a first aspect, the present invention provides a method for inhibiting ovulation in a female subject, comprising administering to said female subject a pharmaceutical composition comprising at least one prostaglandin transporter (PGT) inhibitor.

According to some embodiments, the at least one PGT inhibitor is selected from the group consisting of: an anion exchange inhibitor and a triarylmethane dye. According to some embodiments, the anion exchange inhibitor is 4,4'-Diisothiocyanatostilbene-2,2'-disulfonic acid (DIDS). According to some embodiments, the triarylmethane dye is Bromocresol Green (BCG).

According to some embodiments, the pharmaceutical composition is administered daily from one to four days prior the mid-cycle. According to some embodiments, the pharmaceutical composition is administered daily during mid-cycle and at least one day thereafter. According to some embodiments, the pharmaceutical composition is administered daily from day 9 of the menstrual cycle to day 14 of the menstrual cycle.

According to some embodiments, the method further comprising measuring LH level, and administering said pharmaceutical composition daily, from the day that the level of LH surges until, and including, the day that LH level drops to baseline.

According to some embodiments, the method further comprising measuring estradiol level and administering said pharmaceutical composition daily, at the day that the level of estradiol surges and at least four consecutive days thereafter.

According to some embodiments, the method further comprising measuring daily the level of estradiol and LH and administering said pharmaceutical composition daily at the day that the level of estradiol surges and until the day that the level of LH drops to baseline.

According to some embodiments, the pharmaceutical composition is in a dosage form selected from the group consisting of: pills, patches, vaginal rings and injections.

According to some embodiments, the ovulation is spontaneous ovulation.

According to some embodiment, the subject is a subject undergoing fertility treatments.

According to some embodiments, the ovulation is spontaneous ovulation and the method is applied in the course of fertility treatments for synchronizing the menstrual cycle as required for obtaining optimal ovulation.

According to another aspect, the preset invention provides a pharmaceutical composition comprising at least one PGT inhibitor and a pharmaceutically acceptable carrier, excipient or diluent for use in inhibiting ovulation in a female subject.

According to some embodiments, the at least one PGT inhibitor is selected from the group consisting of: an anion exchange inhibitor and a triarylmethane dye. According to some embodiments, the anion exchange inhibitor is 4,4'-diisothiocyanatostilbene-2,2'-disulfonic acid (DIDS). According to some embodiments, the triarylmethane dye is bromocresol green (BCG).

According to some embodiments, the pharmaceutical composition is for use daily from one to four days prior the mid-cycle. According to some embodiments, the pharmaceutical composition is for use daily during mid-cycle and at least one day thereafter. According to some embodiments, the pharmaceutical composition is for use daily from day 9 of the menstrual cycle to day 14 of the menstrual cycle.

According to some embodiments, the pharmaceutical composition is for use daily, from the day that the level of LH surges until, and including, the day that LH level drops to baseline.

According to some embodiments, the pharmaceutical composition is for use daily, at the day that the level of estradiol surges and at the at least four consecutive days thereafter.

According to some embodiments, the pharmaceutical composition is for use daily at the day that the level of estradiol surges and until the day that the level of LH drops to baseline.

According to some embodiments, the ovulation is spontaneous ovulation. According to some embodiments, the use is for suppressing spontaneous ovulation during fertility treatments.

According to some embodiments, the pharmaceutical composition is formulated in a dosage form selected from the group consisting of: pills, patches, vaginal rings, and injections.

According to yet another aspect, the present invention provides a kit for inhibiting ovulation in a female subject, comprising a container containing a pharmaceutical composition comprising at least one PGT inhibitor; and written instructions for use of said pharmaceutical composition in inhibiting ovulation. According to some embodiments, the kit further comprising means for collecting urine from said female subject. According to some embodiments, the kit further comprising means for measuring LH levels. According to some embodiments, the kit further comprising means for measuring estradiol levels.

According to yet another aspect, the present invention provides use of at least one PGT inhibitor method for inhibiting ovulation in a female subject.

According to yet another aspect, the present invention provides use of at least one PGT inhibitor method for the preparation of a medicament for inhibiting ovulation in a female subject.

Further embodiments and the full scope of applicability of the present invention will become apparent from the detailed description given hereinafter.

### BRIEF DESCRIPTION OF THE DRAWINGS

**Figure 1A** is a bar graph demonstrating PGT mRNA expression in Cumulus Granulosa Cells (CGCs) of expanded post-ovulatory MII COCs (CCM2) and in Cumulus Granulosa Cells (CGCs) of compact GV COCs (CCGV) following IVF treatment.
**Figure 1B** is a bar graph demonstrating PGT mRNA expression in Cumulus Granulosa Cells (CGCs) and Mural Granulosa Cells (MGCs).
**Figure 1C** is a bar graph demonstrating induction of PGT expression in a follicle size-dependent manner.
**Figure 1D** is a bar graph demonstrating PGT mRNA expression in CGCs isolated from mature cumulus oocyte complexes (COCs) (MII), germinal vesicle (GV) and MI COCs.
**Figure 1E** is a bar graph demonstrating PGT mRNA expression in control MGCs or MCGs treated with hCG.
**Figure 1F** presents immunofluorescence images of control MGCs culture (left panel) and MCGs treated with hCG (right panel).
**Figure 1G** presents PGT expression in human GCs following treatment with AREG.
**Figure 2A** is a histological section of ovaries of normally cycling subjects subjected to immunohistochemical staining which presents the expression of the PGT protein in GCs of small antral pre-ovulatory follicles, according to some embodiments.
**Figure 2B** is a histological section of ovaries of normally cycling subjects subjected to immunohistochemical staining which presents the expression of the PGT protein in GCs of small antral pre-ovulatory follicles, according to some embodiments.
**Figure 2C** is a histological section of ovaries of normally cycling subjects subjected to immunohistochemical staining which presents the expression of the PGT protein in GCs of small antral pre-ovulatory follicles, according to some embodiments.
**Figure 2D** is a histological section of ovaries of normally cycling subjects subjected to immunohistochemical staining which presents the expression of the PGT protein in GCs of post-ovulatory follicles.
**Figure 2E** is a histological section of ovaries of normally cycling subjects subjected to immunohistochemical staining which presents the expression of the PGT protein in GCs of post-ovulatory follicles.
**Figure 2F** is a histological section of ovaries of normally cycling subjects subjected to immunohistochemical staining which presents the expression of the PGT protein in GCs of post-ovulatory follicles.
**Figure 2G** is a histological section of ovaries of normally cycling subjects subjected to immunohistochemical staining which presents the expression of the PGT protein in the corpus luteum.
**Figure 2H** is a histological section of ovaries of normally cycling subjects subjected to immunohistochemical staining which presents the expression of the PGT protein in the corpus luteum.
**Figure 2I** is a histological section of ovaries of normally cycling subjects subjected to immunohistochemical staining which presents the expression of the PGT protein in the corpus luteum.
**Figure 3A** is a bar graph demonstrating induction of PGT mRNA expression is in human MGCs by hCG, Forskolin (FSK), myristate acetate (PMA) and the combination of FSK and PMA.
**Figure 3B** is a bar graph demonstrating inhibition of hCG-induced and FSK-induced PGT mRNA expression by the PKA inhibitor H89.
**Figure 3C** is a bar graph demonstrating inhibition of FSK- and PMA-induced PGT mRNA expression by the MEK inhibitor U0126 but not by PI3K inhibitor LY294002.
**Figure 3D** is a bar graph demonstrating inhibition of hCG-induced PGT mRNA expression by the MEK inhibitor U0126.
**Figure 4A** is a bar graph showing Prostaglandin E₂ (PGE₂) levels in the media of MGCs following induction by hCG (by ~2 fold) in the presence or absence of BCG.
**Figure 4B** is a line graph showing PGT upregulation by hCG in association with cellular H³-PGE2 uptake.
**Figure 5A** is a bar graph showing whole ovarian PGT transcripts 0 to 12 hrs after PMSG administration.
**Figures 5B and 5C** are histological micrographs showing cross sections of mice ovaries treated with PMSG.
**Figure 5D** is a histological micrograph showing cross sections of mice ovaries treated with hCG.
**Figure 5E** is a histological micrograph showing cross sections of mice ovaries treated with hCG + DIDS.
**Figures 5F** is a histological micrograph showing cross sections of control (untreated) mice ovaries.
**Figure 5H** is a histological micrograph showing cross sections of ovaries of mice treated with hCG+DIDS.
**Figures 5J** is a histological micrograph showing cross ovaries of mice 40 hours post treatment with hCG.
**Figure 5L** is a histological micrograph showing cross sections of mice 40 hours post treatment with hCG+DIDS.
**Figure 5M** is an ovarian photomicrograph taken 16 hours after the administration of hCG.
**Figure 5N** is an ovarian photomicrograph taken 16 hours after the administration of hCG + BCG.
**Figure 5O** is an ovarian photomicrograph at high magnification taken 16 hours after the administration of hCG.
**Figure 5P** is an ovarian photomicrograph at high magnification taken 16 hours after the administration of hCG + BCG.
**Figure 6A** is a bar graph showing the effect of PGT inhibition by DIDS on hCG upregulation of amphiregulin (Areg).
**Figure 6B** is a bar graph showing the effect of PGT inhibition by DIDS on hCG upregulation of epiregulin (Ereg).
**Figure 6C** is a bar graph showing the effect of PGT inhibition by DIDS on hCG upregulation of tumor necrosis factor alpha-induced protein (TNFAIP6).
**Figure 6D** is a bar graph showing the effect of PGT inhibition by DIDS on hCG upregulation of progesterone receptor (PR).
**Figure 6E** is a bar graph showing the effect of PGT inhibition by DIDS on hCG upregulation of a disintegrin and metalloproteinase with thrombospondin motifs 1 (ADAMTS1).
**Figure 6F** is a bar graph showing the effect of PGT inhibition by DIDS on hCG upregulation of cathepsin L (CTSL).
**Figure 6G** is a bar graph showing the effect of PGT inhibition by DIDS on hCG upregulation of steroidogenic acute regulatory protein (StAR).
**Figure 6H** is a bar graph showing the effect of PGT inhibition by DIDS on hCG upregulation of cholesterol side cleavage chain (p450SCC).
**Figure 6I** is a bar graph showing the effect of PGT inhibition by DIDS on hCG upregulation of follicle stimulating hormone receptor (FSHR).
**Figure 6J** is a bar graph showing the effect of PGT inhibition by DIDS on hCG upregulation of luteinizing hormone receptor (LHR).
**Figure 7A** is a bar graph showing the effect of PGT inhibition by DIDS on hCG upregulation of prostaglandin EP2 receptor (EP2).
**Figure 7B** is a bar graph showing the effect of PGT inhibition by DIDS on hCG upregulation of prostaglandin EP4 receptor (EP4).
**Figure 7C** is a bar graph showing the effect of PGT inhibition by DIDS on hCG upregulation of PGT.
**Figure 7D** is a bar graph showing the effect of PGT inhibition by DIDS on hCG upregulation of COX-2.
**Figure 7E** is a bar graph showing the effect of PGT inhibition by DIDS on hCG upregulation of prostaglandin dehydrogenase (PDGH).
**Figure 8** is a bar graph showing serum progesterone levels in PMSG-primed/hCG-triggered mice in the absence or presence of DIDS.
**Figure 9A** is a bar graph showing intracellular cAMP in cells stimulated by PGE₂.
**Figure 9B** is a bar graph showing EP4 receptor mRNA expression in cells stimulated by PGE₂.

### DETAILED DESCRIPTION OF THE INVENTION

The present invention provides novel non hormonal based contraceptive compositions. The present invention further provides the use of a prostaglandin transporter (PGT) specific inhibitor, for preventing the process of ovulation. Thus, according to the present invention PGT inhibitors are effective contraceptives.

Birth controls and fertility controls are contraceptive methods and devices used to prevent pregnancy. The most effective birth controls are sterilization by means of vasectomy in males and tubal ligation in females, intrauterine devices (IUDs) and implantable contraceptives, but these methods are non-reversible in essence. Reversible methods of contraception include hormonal contraceptives (e.g., oral pills, patches, vaginal rings, and injections) and barriers (e.g., condoms, diaphragms, and contraceptive sponge). Hormonal contraceptives are commonly associated with side effects such as headaches, depression and other mood changes, nausea and vomiting, breast tenderness, breakthrough bleeding or spotting, decreased enjoyment of sex and weight gain. Some side effects are uncommon but may be dangerous especially for smoking women, those include heart attack, an increase in systolic and diastolic blood pressure, migraines and blood clots. Furthermore, oral contraceptives must be taken every day for months, and hence use thereof is typically associated with poor patient compliance. Barriers contraceptives are mostly less effective as compared to hormonal contraceptives and are associated with discomfort.

The contraceptive compositions of the present invention are devoid of hormones and the side effects associated therewith and do not cause any discomfort compared to barrier contraceptives. Thus, the contraceptive compositions of the present invention confer an advantage over known contraceptive approaches. Furthermore, the contraceptive platform of the present invention may be used only during a particular short period of the menstrual cycle, and thus provides advantageous treatment regimen and patient compliance over the contraceptives known to date.

According to some embodiments, the present invention provides a method for inhibiting ovulation in a female subject, comprising administering to said female subject a pharmaceutical composition comprising at least one prostaglandin transporter (PGT) inhibitor.

PG transporter (PGT) is a functional PG carrier with high affinity for PGE2, PGF2α and PGD2. PGT belongs to the super family of 12-transmembrane organic anion transporting polypeptides (OATPs) and has been identified in rats, humans, mice and bovine PGT. It is to be understood that the term "PGT" is interchangeable with any alternative name or synonyms of this protein known in the art including, but not limited to: solute carrier organic anion transporter family, member 2A1, human (SLCO2A1).

As used herein, the term "PGT inhibitor" refers to any one or more inhibitors capable of inhibiting PGT or PGT associated activity. According to some embodiments, the PGT inhibitor is capable of inhibiting at least one of: oocyte nuclear maturation, cumulus expansion, ovulation and follicular rupture. According to some embodiments, the PGT inhibitor is an inhibitor capable of inhibiting PGT expression, translation and/or activity. According to some embodiments, the PGT inhibitor is selected from the group consisting of: a chemical agent or moiety, a protein, a polypeptide or a peptide, and a polynucleotide molecule. Each possibility represents a separate embodiment of the invention.

As used herein, the phrase "inhibiting PGT activity" comprises any one or more of the following: attenuating, reducing or preventing cellular processes, pathways or phenotypes associated with PGT. Each possibility represents a separate embodiment of the invention.

According to some embodiments, inhibiting PGT activity is mediated by at least one of: reducing, inhibiting, preventing or neutralizing the functionality of PGT, inducing PGT's desensitization and inducing PGT's internalization. Each possibility represents a separate embodiment of the invention.

According to some embodiments, inhibiting PGT activity comprises any one or more of inhibiting oocyte nuclear maturation, inhibiting cumulus expansion, inhibiting ovulation, inhibiting follicular rupture, inhibiting or suppressing spontaneous ovulation and inhibiting prostaglandins (PGs) efflux. Each possibility represents a separate embodiment of the invention.

As used herein, the term "inhibiting ovulation" refers to, but is not limited to, any one or more of the following: abrogating, blocking, halting, attenuating or preventing the process, onset or event of ovulation. Each possibility represents a separate embodiment of the invention. According to some embodiment, this term includes, and is not limited to, administration of a PGT inhibitor to a female subject in order to prevent ovulation in said female subject.

As used herein the term "ovulation" refers to the event or process of a female's menstrual cycle in which an egg (ovule) is released from the ovaries to the uterus. Ovulation occurs in mid-cycle, approximately 24- 36 hours following a surge of luteinizing hormone (LH). In humans, the few days surrounding ovulation (from approximately days 10 to 18 of a 28 day cycle) constitute the most fertile phase. On average, the time from the beginning of the last menstrual period (LMP) until ovulation is 14.6 days, but with substantial variation between women and between cycles in any single woman, with an overall 95% prediction interval of 8.2 to 20.5 days.

According to some embodiments, inhibiting PGT expression comprises inhibiting production of an end-product e.g., PGT mRNA or PGT protein. Each possibility represents a separate embodiment of the invention.

According to some embodiments, inhibiting PGT expression comprises reducing the expression of PGT by at least 15%, 20% 30%, 40%, 50%, 60%, 70%, 80%, 90%, or any amount of reduction in between the specifically recited percentages, as compared to the levels of PGT expression in the absence of PGT inhibitor(s).

According to some embodiments, the PGT inhibitor is a chemical agent or moiety selected from the group consisting of: an anion exchange inhibitor, a triarylmethane dye and a compound as disclosed in US patent No. 8,227,466. Each possibility represents a separate embodiment of the invention.

According to some embodiments, the anion exchange inhibitor is 4,4'-Diisothiocyanatostilbene-2,2'-disulfonic acid (DIDS) or a derivative or an analogue thereof.

According to some embodiments, the triarylmethane dye is Bromocresol Green (BCG) a derivative or an analogue thereof.

According to some embodiments, the chemical agent or moiety is a compound of formula I:

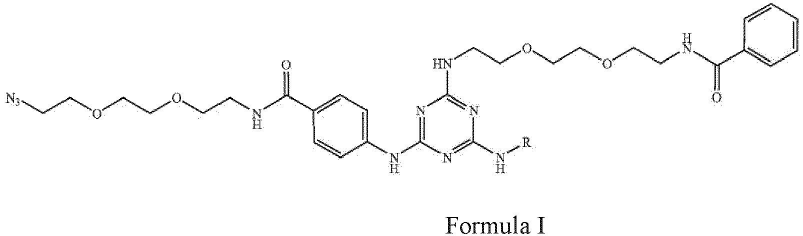

According to some embodiments, R is a C₁-C₁₅ straight or branched alkyl, a substituted alkyl, a cycloalkyl, a carboxyalkyl, a substituted cycloalkyl, a C₁-C₁₅ straight or branched alkenyl, a substituted alkenyl, a cycloalkenyl, a substituted cycloalkenyl, a C₁-C₁₅ straight or branched alkinyl, a substituted alkinyl, a cycloalkinyl, a substituted cycloalkinyl, a C₁-C₁₀ straight or branched ether, a substituted ether, a cycloether, an ester, an amide, an acetyl, an aminal, an anhydride, an aryl, a substituted aryl, a heteroaryl, a substituted heteroaryl, a carboxyaryl, a heterocyclic group, a substituted heterocyclic group, a fused cycloalkyl, a substituted fused cycloalkyl, a fused heterocyclic group, a substituted fused heterocyclic group, a fused aryl, a substituted fused aryl, a fused heteroaryl, a substituted fused heteroaryl ring, or any combination thereof, optionally further comprising a hydroxy, an alkoxy, an aryloxy, an oxo, an ester, an ether, an amine, an azo, an azido, a nitro, an imine, an isothionate, a carbonyl, a peroxide, a halogen, a formyl, an acyl, a carboxy, an amido, a carbamoyl, a guanidino, a ureido, a amidino, a thiol, a mercapto, a sulfinyl, a sulfonyl and/or a sulfonamide. According to some embodiments, R comprises a carboxyl or phenol group.

As used herein the term "an analogue or a derivative thereof' refers to suitable active variants of the PGT inhibitors described herein, such as, an analog or a modified molecule. Chemical modification, in the context of the present invention includes modification with a chemical entity, group or moiety. Moreover, each particular compound, such as those described herein, may give rise to an entire family of analogues or derivatives having similar activity and, therefore, usefulness according to the present invention. Likewise, a single compound, such as those described herein, may represent a single family member of a greater class of compounds useful according to the present invention. Accordingly, the present invention fully encompasses not only the compounds described herein, but analogues and derivatives of such compounds, particularly those identifiable by methods commonly known in the art and recognizable to the skilled artisan.

According to some embodiments, the polynucleotide molecule is a nucleic acid sequence or a molecule capable of hybridizing to nucleic acids encoding or controlling PGT expression. Exemplary nucleic acid sequences suitable in the context of the present invention include, but are not limited to, an RNA interference (RNAi) molecule, small hairpin RNA (shRNA) or a small interference RNA (siRNA) molecule, a micro RNA (miRNA) molecule and an antisense molecule. Each possibility represents a separate embodiment of the invention.

According to yet another embodiment, the PGT inhibitor is a protein, a polypeptide or a peptide. Each possibility represents a separate embodiment of the invention. The protein, polypeptide or peptide may be a synthetic or a recombinant protein, polypeptide or peptide. The PGT inhibitor may be a chimeric or fusion protein, polypeptide or peptide composed of at least two portions of a protein, a polypeptide or a peptide.

According to some embodiments, the method further comprises measuring LH level, and administering said pharmaceutical composition daily, from the day that the level of LH surges until, and including, the day that LH level drops to baseline.

According to some embodiments, the method further comprises measuring estradiol level and administering said pharmaceutical composition daily, at the day that the level of estradiol surges and at the at least four consecutive days thereafter.

According to some embodiments, the method further comprises measuring daily the level of estradiol and LH and administering said pharmaceutical composition daily at the day that the level of estradiol surges and until the day that the level of LH drops to baseline.

According to some embodiments, there is provided a pharmaceutical composition comprising a therapeutically effective amount of at least one prostaglandin transporter (PGT) inhibitor for inhibiting ovulation in a female subject.

As used herein, the term "therapeutically effective amount" refers to an amount of one or more PGT inhibitor(s) which is effective in inhibiting or preventing ovulation.

The present invention provides birth control or contraceptive compositions for a female subject. The subject may be a female in need of regular contraception. The female subject may be a mammal, particularly, human.

The methods of the invention are useful for female subjects during their menstrual cycle, commonly, female who reached puberty. The methods remain useful up until menopause (commonly considered the end of a female's reproductive life). In humans, female's reproductive life usually begins around the age of about 12 and ends around the age of 50.

As used herein the term "menstrual cycle" refers to the process responsible for the production of eggs, and the preparation of the uterus for pregnancy.

Typically in humans and primates, the count of an individual menstrual cycle in days starts at the first day of menstrual bleeding. Stimulated by gradually increasing amounts of estrogen in the follicular phase, discharges of blood (menses) slow then stop, and the lining of the uterus thickens. Follicles in the ovary begin developing under the influence of a complex interplay of hormones, and after several days one or occasionally two become dominant (non-dominant follicles atrophy and die). Approximately mid-cycle, 24 - 36 hours after the Luteinizing Hormone (LH) surges, the dominant follicle releases an ovum, or egg, in an event called ovulation. After ovulation, the egg only lives for 24 hours or less without fertilization while the remains of the dominant follicle in the ovary become a corpus luteum; this body has a primary function of producing large amounts of progesterone. Under the influence of progesterone, the endometrium (uterine lining) changes to prepare for potential implantation of an embryo and establish pregnancy. If implantation does not occur within approximately two weeks, the corpus luteum will involute, causing sharp drops in levels of both progesterone and estrogen. The hormone drop causes the uterus to shed its lining and egg in a process termed menstruation.

As demonstrated herein below, PGT expression in the ovary is induced by hCG. hCG is an analogue of luteinizing hormone (LH) used to induce ovulation in the clinic and in animals. Ovulation typically occurs in the mid-cycle of women's menstrual cycle and follows a surge of LH which is preceded by a gradual elevation of estradiol. Thus, the PGT inhibitors of the invention are particularly useful when administered during or a few days prior to the mid-cycle. According to some embodiments, the PGT inhibitor, or a pharmaceutical composition comprising same, is administered 6 days, 5 days, 4 days, 3 days, 2 days, or 1 day prior to the mid-cycle. Each possibility represents a separate embodiment of the invention.

According to some embodiments, the at least one PGT inhibitor is administered during the middle of the menstrual cycle, also termed herein "mid cycle" or "mid-cycle period". According to some embodiments, administration of the PGT inhibitor is synchronized with the level of estradiol and LH. According to some embodiments, administration of the PGT inhibitor is synchronized with the level of E3G, the urinary metabolite of estradiol, and LH.

As used herein, the terms "estradiol" and "E3G" are interchangeable.

According to some embodiments, the at least one PGT inhibitor is administered as LH level surges. According to some embodiments, the at least one PGT inhibitor is administered as LH levels surges and on the following day. According to some embodiments, the at least one PGT inhibitor is administered daily, beginning when estradiol levels start to build up and continuing until the level of LH drops to threshold level. According to some embodiments, the at least one PGT inhibitor is administered daily, for approximately six days, as follows: about two to three days prior to the surge of LH, 1-2 days during which the level of LH is high, following a day or two. According to some embodiments, the at least one PGT inhibitor is administered following LH surge. In accordance with this embodiment, the method of the invention further comprises a step of detecting LH, and, optionally, estradiol and/or E3G, with suitable detection methods or kits available in the market, such as, ovulation kits. According to some embodiment, the method of the invention further comprises a step of detecting LH, and, optionally, E3G, in the urine of said female subject, prior to said administering step.

According to some embodiments, the pharmaceutical composition is administered daily from one to four days prior the mid-cycle. According to some embodiments, the pharmaceutical composition is administered daily during mid-cycle and at least one day thereafter. According to some embodiments, the pharmaceutical composition is administered daily from day 9 of the menstrual cycle to day 14 of the menstrual cycle.

According to some embodiments, the invention provides a "once-a-month contraceptive", or a "once in a menstrual cycle contraceptive", which is administered once a month on a single occasion, every month, more precisely on a single occasion during each menstrual cycle. According to some embodiments, the contraceptive is administered several times during the menstrual cycle, for example, on each day surrounding the, or prior to, and optionally following, the mid-cycle or the event of LH surge.

According to some embodiments, the subject in need thereof is a woman having a regular menstrual cycle, e.g. a 28, 29, 30, or 31 days cycle.

In case the woman has a regular menstrual cycle, the PGT inhibitor may be administered daily during the 9th and the 14^{th} days of the menstrual cycle. According to some embodiments, the PGT inhibitor may be administered daily during the 10th and the14th days, the 11th and the 14th days, the 12th and the 14^{th} days, or the 13th and the 14th days of the menstrual cycle. Each possibility represents a separate embodiment of the invention. According to some embodiments, the PGT inhibitor may be administered on the 14^{th} of the menstrual cycle. Of note, the 1^{st} day of the women menstrual cycle is the first day of her period.

The methods of the present invention are further useful for suppressing spontaneous ovulation during fertility treatments, including, but not limited to, *in vitro* fertilization (IVF). The methods may be applied in order to synchronize the menstrual cycle and/or as part of controlled ovarian hyperstimulation protocol utilized in fertility treatments.

The term "fertility treatments" is interchangeable with the term "assisted reproduction technique" and includes, but is not limited to, in vitro fertilization (IVF).

Controlled ovarian hyperstimulation typically involves use of fertility agents (commonly analogues of follicle-stimulating hormone (FSH) and/or human Menopausal Gonadotrophins (hMG)) to induce ovulation by multiple ovarian follicles. These multiple follicles may be isolated by oocyte retrieval, e.g. for IVF, or be given time to ovulate, resulting in superovulation which is the ovulation of a larger-than-normal number of eggs, generally in the sense of at least two. When used in conjunction with an assisted reproductive technique (such as IVF), controlled ovarian hyperstimulation confers a need to suppress spontaneous ovulation. In this respect, the PGT inhibitors of the present invention may be utilized for suppressing spontaneous ovulation.

To date, ovulation suppression may be achieved by either Gonadotropin-releasing hormone (GnRH) agonist or by GnRH antagonist administration. Those treatments are hormonal based and may be associated with side effects. Thus, the methods of the present invention are useful for suppressing spontaneous ovulation in an assisted reproduction technique in a safer manner than the current GnRH based treatments.

According to some embodiments, there is provided a method for inhibiting the expression of one or more oocyte maturation genes, comprising administering to a subject in need thereof a pharmaceutical composition comprising at least one PGT inhibitor.

According to some embodiments, said inhibiting the expression of one or more oocyte maturation genes comprises inhibiting hCG induced expression of said one or more oocyte maturation genes.

According to some embodiments, said one or more oocyte maturation genes may include amphiregulin (AREG), epiregulin (EREG) and/or progesterone receptor (PR). Each possibility represents a separate embodiment of the invention.

According to some embodiments, there is provided a method for inhibiting the expression of one or more cumulus expansion genes, comprising administering to a subject in need thereof a pharmaceutical composition comprising at least one PGT inhibitor.

According to some embodiments, said inhibiting the expression of one or more cumulus expansion genes comprises inhibiting hCG-induced expression of said one or more cumulus expansion genes.

According to some embodiments, said one or more cumulus expansion genes may include tumor necrosis factor alpha-induced protein (TNFAIP6).

According to some embodiments, there is provided a method for inhibiting the expression of one or more follicular rupture related genes, comprising administering to a subject in need thereof a pharmaceutical composition comprising at least one PGT inhibitor.

According to some embodiments, said inhibiting the expression of one or more follicular rupture related genes comprises inhibiting hCG-induced expression of said one or more follicular rupture related genes.

According to some embodiments, said one or more follicular rupture related genes may include disintegrin, metalloproteinase with thrombospondin motifs 1 (ADAMTS1) and/or cathepsin L (CTSL). Each possibility represents a separate embodiment of the invention.

According to some embodiments, there is provided a method for inhibiting the expression of one or more steroidogenesis related genes, comprising administering to a subject in need thereof a pharmaceutical composition comprising at least one PGT inhibitor.

According to some embodiments, said inhibiting the expression of one or more steroidogenesis related genes comprises inhibiting hCG-induced expression of said one or more steroidogenesis related genes.

According to some embodiments, said one or more steroidogenesis related genes may include steroidogenic acute regulatory protein (StAR) and/or cholesterol side cleavage chain (p450SCC). Each possibility represents a separate embodiment of the invention.

According to some embodiments, there is provided a method for inducing the expression of one or more ovulatory related genes, comprising administering to a subject in need thereof a pharmaceutical composition comprising at least one PGT inhibitor.

According to some embodiments, said one or more ovulatory related genes may include follicle stimulating hormone receptor (FSHR) and/or luteinizing hormone/chorionic gonadotropin receptor (LHCGR). Each possibility represents a separate embodiment of the invention.

According to some embodiments, there is provided a method for inducing the expression of EP2, comprising administering to a subject in need thereof a pharmaceutical composition comprising at least one PGT inhibitor. According to some embodiments, said inducing the expression of EP2 comprises inducing the expression of hCG- suppressed EP2.

According to some embodiments, there is provided a method for inhibiting the expression of any one or more of EP2, COX-2 and progesterone, comprising administering to a subject in need thereof a pharmaceutical composition comprising at least one PGT inhibitor. Each possibility represents a separate embodiment of the invention. According to some embodiments, said inhibiting

As used herein a "pharmaceutical composition" refers to a preparation of one or more of the PGT inhibitor(s) described herein, with other components, such as pharmaceutically inactive components, including, pharmaceutically acceptable carriers and excipients. Commonly, the purpose of a pharmaceutical composition is to facilitate administration of a compound to a subject.

As used herein, the term "excipient" refers to an inert substance added to a pharmaceutical composition to further facilitate administration of the pharmaceutical active compound, namely, the PGT inhibitor. Examples, without limitation, of typical excipients include calcium carbonate, calcium phosphate, various sugars and types of starch, cellulose derivatives, gelatin, vegetable oils, and polyethylene glycols. Each possibility represents a separate embodiment of the invention.

As used herein the phrase "pharmaceutically acceptable carrier" refers to a carrier, an excipient or a diluent that does not cause significant irritation to an organism and does not abrogate the biological activity and properties of the pharmaceutical active compound.

The term "carrier" refers to any substance suitable as a vehicle for delivering of the PGT inhibitor of the present invention to a suitable biological site or tissue. As such, carriers can act as a pharmaceutically acceptable excipient of the pharmaceutical composition of the present invention.

The PGT inhibitor may be administered by any convenient route, including oral, buccal, sublingual, parenteral (e.g., intramuscular, transdermal), vaginal (for example in the form of a gel, or a foam), gingival, nasal, rectal, etc. Each possibility represents a separate embodiment of the invention.

The PGT inhibitor may be administered in any suitable dosage form, including but not limited to, pills, patches, vaginal rings, foam, gel, injections and the alike. Each possibility represents a separate embodiment of the invention.

According to some embodiments, the pharmaceutical composition comprising said at least one PGT inhibitor is an oral dosage form. According to some embodiments, said pharmaceutical composition is administered per os.

According to some embodiments, the pharmaceutical composition comprising said at least one PGT inhibitor is an immediate-release formulation. Namely, the formulation comprising the PGT inhibitor is based on the rapid effect of the PGT inhibitor before the ovulation.

The effective dose, also termed herein "therapeutically effective amount" of the at least one PGT inhibitor may be determined by a person of skill in the art while taking into consideration various conditions of the subject, for example, general health conditions of the subject, age and weight.

According to some embodiments, the PGT inhibitor is an anion exchange inhibitor. According to some embodiments, the anion exchange inhibitor is DID. According to some embodiments, the DID is administered in a dose of between 25 to 100 mg/kg.

According to some embodiments, the anion exchange inhibitor is a triarylmethane dye. According to some embodiments, the anion exchange inhibitor is BCG. According to some embodiments, the triarylmethane dye is administered in a dose of between 350 to 1000 mg/kg.

The frequency of administration depends on the properties of the PGT inhibitor and the conditions of the subject and may be, for example, a plurality of times a day (i.e. 2, 3, 4, 5, or more times per day), or once a day. Each possibility represents a separate embodiment of the invention.

According to some embodiments, there is provided a kit for inhibiting ovulation, comprising a pharmaceutical composition comprising at least one PGT inhibitor and a carrier; and instruction for use of said pharmaceutical composition for inhibiting ovulation.

According to some embodiments, the kit is for inhibiting spontaneous ovulation during fertility treatments.

According to some embodiments, the PGT inhibitor is for administration in proximity with LH surge.

In accordance with those embodiments, the kit further comprises means for detecting LH surge. Exemplary means may include antibodies capable of detecting LH in the urine of a subject. According to some embodiments, the kit comprises one or more vessels for collecting a urine sample of the subject.

According to some embodiments, the kit further comprises means for measuring LH level. According to some embodiments, the instructions for use indicate to use the at least one PGT inhibitor in proximity with LH surge. According to some embodiments, the instructions for use indicate to measure LH level daily and to use the at least one PGT inhibitor in proximity with LH surge and at least one day after. According to some embodiments, the instructions for use indicate to measure LH level daily, starting from day 5 of the menstrual cycle and up to the decrease of LH level to baseline, and to use the at least one PGT inhibitor in proximity with LH surge and at least one day after.

According to some embodiments, the baseline may be the level of LH in any one or more of the days from day 1 through day 8 and from day 15 to day 28, of the menstrual cycle. According to some embodiments, the baseline level of LH is the average of the daily level of LH in two or more days during day 1 through day 8 of the menstrual cycle and day 15 to day 28 of the menstrual cycle.

According to some embodiments, the kit further comprises means for detecting estradiol level. According to some embodiments, the instructions for use indicate to use the at least one PGT inhibitor upon an increase in estradiol level and during the 6 days following the increase. According to some embodiments, the instructions for use indicate to measure estradiol and LH level daily, starting from day 5 of the menstrual cycle and up to the decrease of LH level to baseline, and to use the at least one PGT inhibitor in proximity with an increase in estradiol level and at least 5 days thereafter.

According to some embodiments, the kit comprises a pack designed to hold exactly a month's worth of dosage forms, e.g. pills, wherein the dosage forms of days 1 through 8 and 15 to 28 are mock pills which do not contain active ingredients, and the dosage forms of days 9 to 14 comprise a pharmaceutical composition comprising at least one PGT inhibitor.

According to some embodiments, the pack is a dispenser pack.

According to some embodiments, the dosage forms of days 9 to 14 are colored differently than the mock dosage forms.

The following examples are presented in order to more fully illustrate some embodiments of the invention.

### EXAMPLES

### EXAMPLE 1 - PGT expression in human granulosa cells

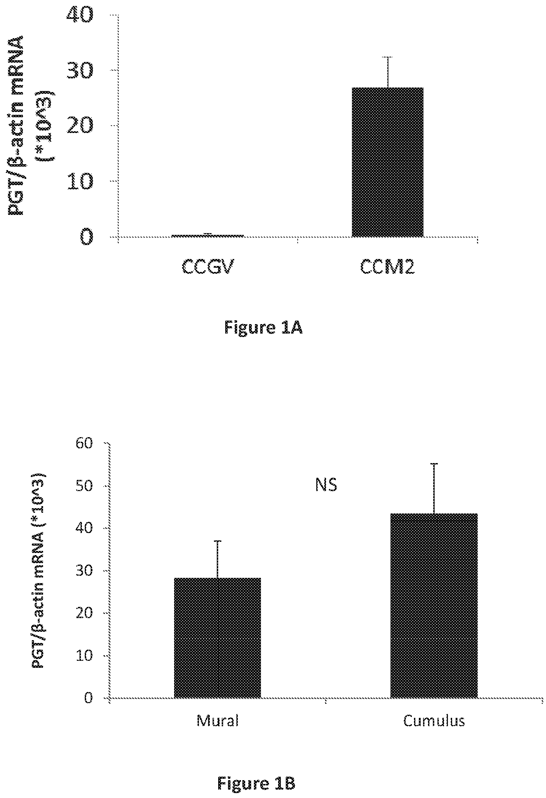

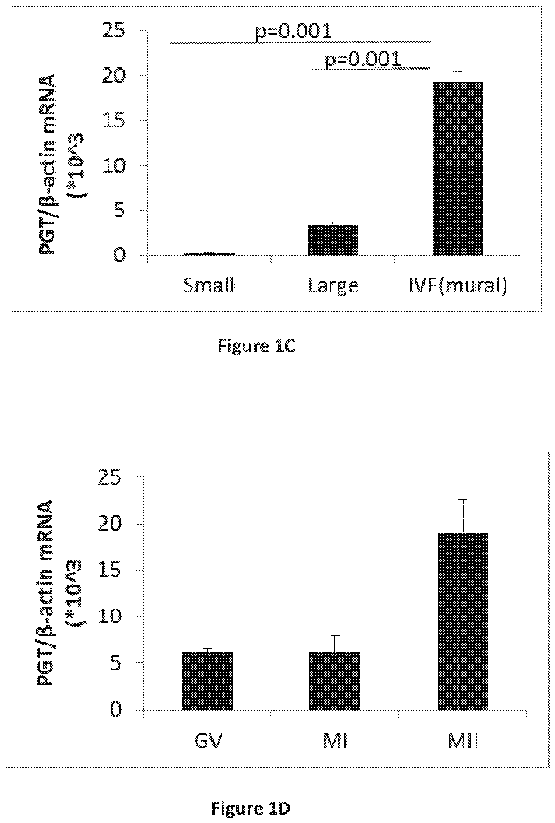

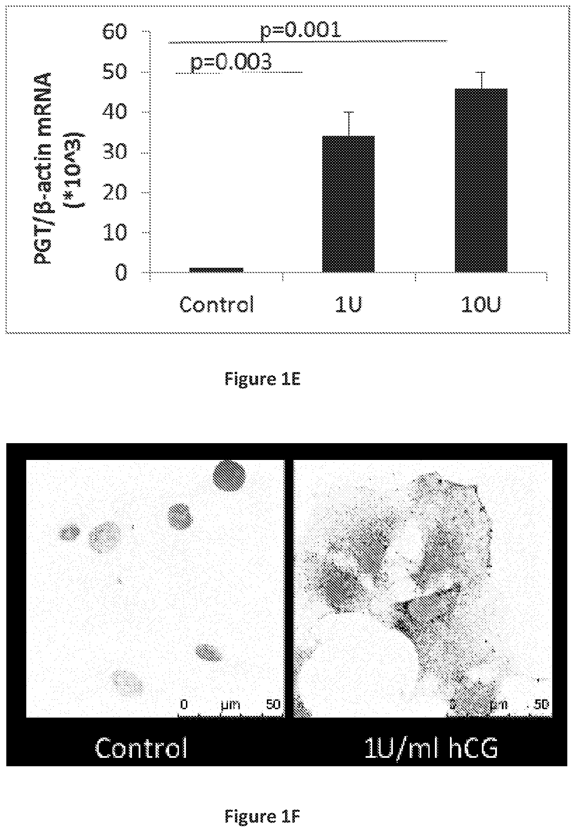

Quantitative PCT (qPCR) analysis was used to validate the ovulation-associated upregulation of PGT transcripts noted with RNAseq. As shown in Fig. 1A, the in vivo expression of PGT transcripts in CGCs of expanded post-ovulatory MII COCs following IVF treatment proved 90-fold higher as compared with CGCs of compact GV COCs following IVF treatment (p<0.01). The in vivo expression of PGT transcripts was also studied in mural GCs (MGCs) of pre-ovulatory (>17 mm) follicles obtained in the course of in vitro fertilization (IVF). As shown in Fig. 1B, CGCs expressed higher levels of PGT transcripts relative to mural counterparts but not to a level of statistical significance (p=0.20). The expression pattern of PGT transcripts during late antral follicular development was assessed using MGCs of small (<10 mm) and large (10-14 mm) in vitromatured (IVM) follicles. Use was also made of MGCs of pre-ovulatory (≥17 mm) follicles obtained in the course of IVF. The expression of PGT transcripts in MGCs increased progressively as a function of follicular size (Fig. 1C, p=0.001). Moreover, the expression of PGT transcripts is related to oocyte maturation, since the expression of PGT transcripts proved significantly (p=0.037) higher in CGCs of mature MII COCs as compared with GV and MI COCs (Fig. 1D). No significant difference was detected in the expression of PGT transcripts between CGCs of GV or MI COCs. Taken together, these observations are consistent with the view that the expression of PGT transcripts in MGCs is progressively upregulated during the final stages of follicular maturation.

### EXAMPLE 2 - PGT expression in cultured human mural granulosa cells

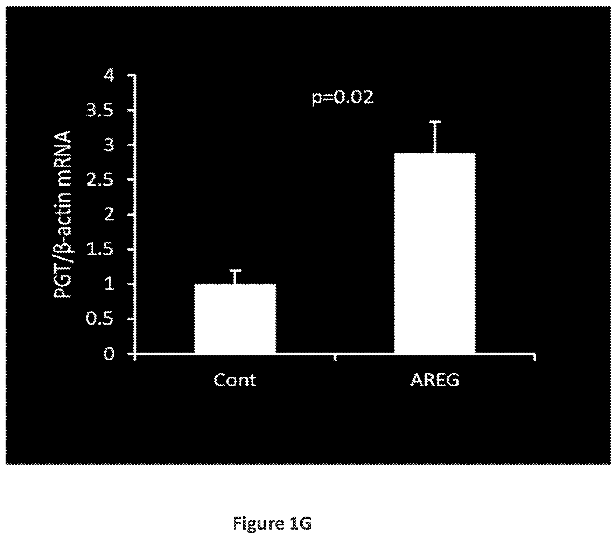

To confirm the effect of LH/CGR (Luteinizing Hormone/Choriogonadotropin Receptor) activation on the expression of PGT transcripts in GCs, primary MGCs that were pretreated for 48 hours with follicle-stimulating hormone (FSH) were subsequently incubated in the absence or presence of hCG. These cells provide an in vitro model of nonluteinized human mural granulosa cell culture to study the effects of LH/hCG as well as other regulatory agents. The FSH-pretreated MGCs (48 hours) were re-incubated with (1 or 10 U/ml) or without hCG for an additional 24 hours. As shown in Fig. IE, treatment of MGCs with hCG resulted in the induction of PGT transcripts at both concentrations (34 and 46-fold respectively, p=0.003 and p=0.001) by this LH receptor ligand. Comparable observations were made at the protein level by immunofluorescence (Fig. 1F). For measuring the effect of amphiregulin (AREG), an LH/hCG inducible factor that regulates resumption of cumulus-oocyte activities, on PGT expression MGCs aspirated during IVF procedures were initially cultured for 4 days followed by FSH stimulation (1U/ml) for 48 hours. FSH-pretreated MGCs were then exposed to vehicle (PBS) or AREG for 24 hours. PGT was quantified by qPCR and normalized to *β-actin* expression. Data represent the mean ± SEM of three independent experiments. The results indicate that AREG significantly induced PGT expression in MCG (3-fold, p=0.02, Fig. 1G).

### EXAMPLE 3 - Spatiotemporal expression of PGT protein in the human ovary

To examine the spatiotemporal expression pattern of the PGT protein in the human ovary, histological sections of the ovaries of normally cycling subjects were subjected to immunohistochemical staining. Staining intensity was evaluated using a scale of 0-3 and the percentage of cells stained in each structure evaluated (Table 1).

**Table 1 - Scoring of PGT immunohistochemical staining**

| Case No. | | Primordial | Primary | Secondary small | Secondary large | Antral small | Antral large | Post ovulatory | Corpus luteum |
|---|---|---|---|---|---|---|---|---|---|
| 1 | Intensity | 0 | 1 | NA | NA | 1 | 2 | 3 | 1 |
| | Stained cells (%) | 0 | 100 | NA | NA | 40 | 80 | 90 | 90 |
| 2 | Intensity | NA | NA | NA | NA | NA | 2 | NA | 1 |
| | Stained cells (%) | NA | NA | NA | NA | NA | 80 | NA | 90 |
| 3 | Intensity | NA | 0 | 0 | 0 | NA | 2 | 3 | NA |
| | Stained cells (%) | NA | 0 | 0 | 0 | NA | 70 | 60 | NA |
| 4 | Intensity | 0 | 0 | NA | NA | NA | NA | NA | 1 |
| | Stained cells (%) | 0 | 0 | NA | NA | NA | NA | NA | 30 |
| 5 | Intensity | 0 | 0 | NA | NA | 0 | 2 | 0 | NA |
| | Stained cells (%) | 0 | 0 | NA | NA | 0 | 70 | 0 | NA |
| 6 | Intensity | NA | NA | NA | NA | NA | NA | NA | 1 |
| | Stained cells (%) | NA | NA | NA | NA | NA | NA | NA | 90 |

| | | | | | | | | | |
|---|---|---|---|---|---|---|---|---|---|
| NA - Not Applicable | | | | | | | | | |

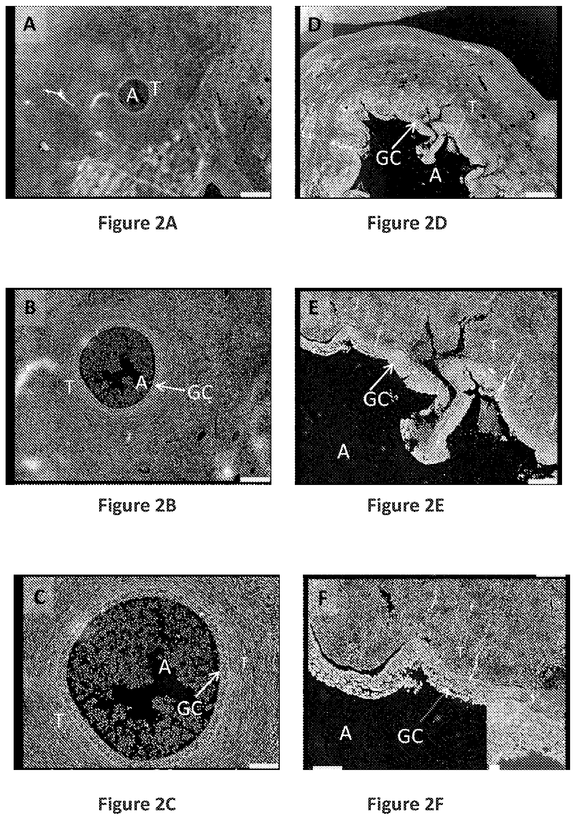

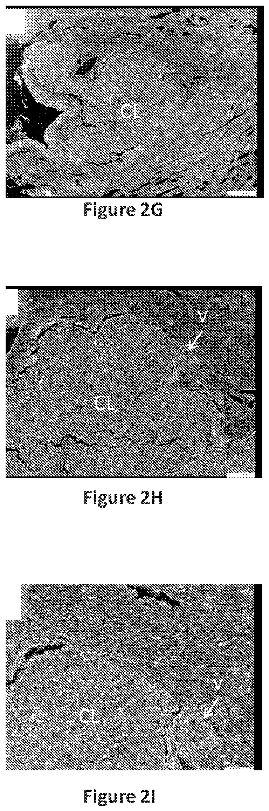

The expression of the PGT protein in GCs is further shown in Figs. 2A-2J, which represent the antrum (A), granulosa cells (GC), theca cells (T), corpus luteum (CL) and luteal blood vessels (V). As shown in Figs. 2A-2C, the expression of the PGT protein in GCs of small antral pre-ovulatory follicles was negligible (Fig. 2A scale bar = 500 µm; Fig. 2B scale bar = 200 µm; Fig. 2C scale bar = 100 µm). In contrast, the expression of the PGT protein in GCs of post-ovulatory follicles (Figs. 2D-2F, scale bars are 500 µm, 200 µm and 100 µm, respectively and scale bar in inset to Fig. 2F is 25 µm) and in the corpus luteum (CL) (Figs. 2G-2I, scale bars are 500 µm, 200 µm and 100 µm, respectively) was markedly increased (Figs. 2D-2I - dark staining). The expression of the PGT protein in theca cells (Fig. 2A-2J; T) was low but detectable throughout the life cycle of the developing antral follicle. These results support the conclusion that ovarian expression of PGT transcripts is ovulation-dependent.

### EXAMPLE 4 -PGT expression in cultured human mural granulosa cells: Intracellular signaling pathways

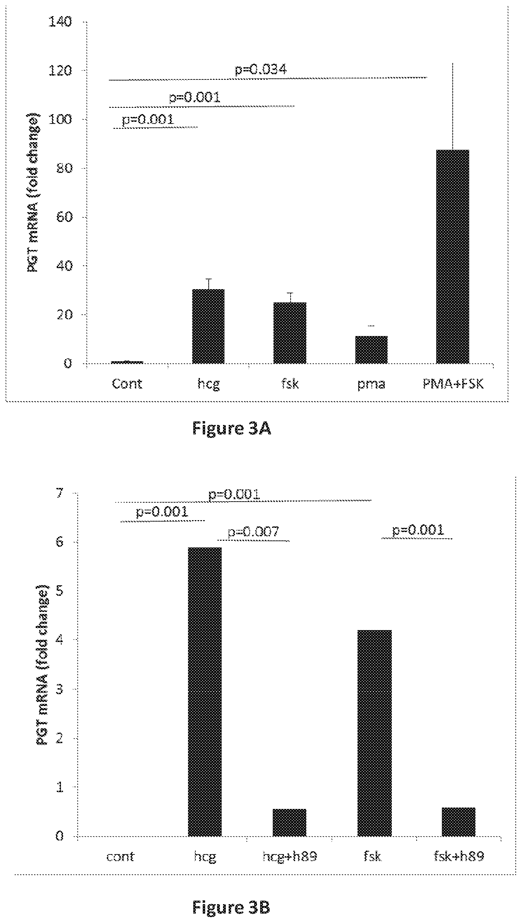

To determine the role of protein kinase A (PKA) and/or protein kinase C (PKC) signaling pathways in the hCG-induced upregulation of PGT expression, FSH-pretreated MGCs (48 hours) were re-incubated with phorbol myristate acetate (PMA; 20nM) and/or forskolin (FSK; 10µM) for an additional 24 hours so as to activate the PKC and PKA signaling pathways, respectively. As shown in Fig. 3A, treatment with FSK, an activator of adenylate cyclase, increased the PGT transcripts in MGCs to levels similar to those effected by hCG (25- and 30-fold induction, respectively, p=0.001). In contrast, treatment with PMA, an activator of the PKC signaling pathway, increased the PGT transcripts in MGCs to a lesser extent (11-fold induction). Concurrent treatment with both FSK and PMA resulted in an 87-fold increase in PGT transcripts thereby suggesting marked synergy between the two signaling pathways (p=0.034). To further explore the role of PKA in the hCG-induced upregulation of PGT expression, FSH-pretreated MGCs (48 hours) were re-incubated initially with or without H89 (a selective PKA inhibitor; 10µM) for 1 hour and then with either of hCG (1U/ml) or of FSK (10µM) for an additional 24 hours. As shown in Fig. 3B, pretreatment with H89 largely inhibited the hCG- and FSK-induced upregulation of PGT expression in MGCs (p=0.01).

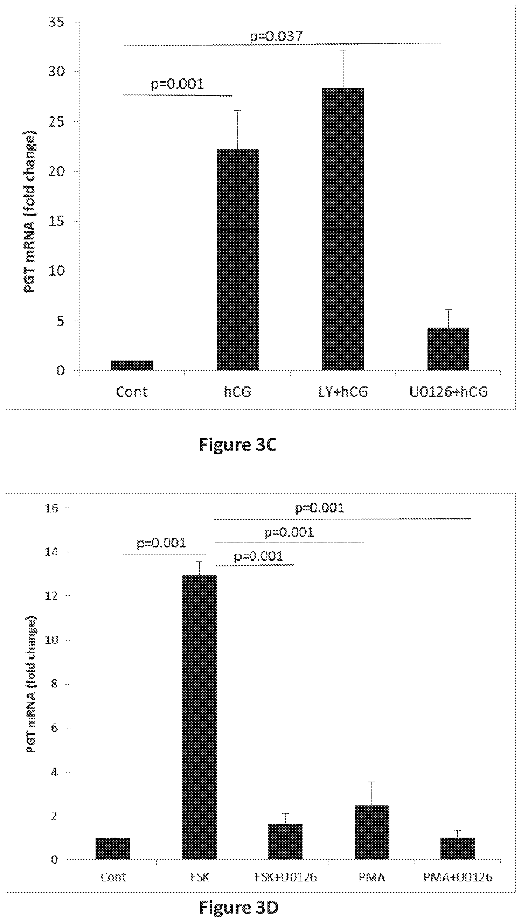

The role of extracellular receptor kinase (ERK)1/2 and PI3K/AKT (putative LHCGR action mediators) in the hCG-induced upregulation of PGT expression was examined in GCs. To this end, FSH-pretreated MGCs (48 hours) were initially pretreated with or without LY294002 (a PI3K inhibitor; 10µM) or U0126 (an inhibitor of MEK, an upstream activator of ERK; 10µM) for 1 hour and then re-incubated with hCG (1U/ml), FSK (10µM), or PMA (20nM) for 24 hours. As shown in Fig. 3C, pretreatment with U0126 effectively abolished the hCG-induced upregulation of PGT transcripts in MGCs (p=0.037). In contrast, pretreatment with LY294002 was without effect. Finally, as shown in Fig. 3D, pretreatment with U0126 markedly inhibited the FSK- and PMA-mediated upregulation of PGT transcripts (p=0.001). These observations suggest that both the PKA and the PKC signaling pathways are involved in the hCG-induced upregulation of PGT expression in MGCs. The ERK/MEK pathway, in turn, appears to be engaged further downstream along the PKA and PKC signaling pathways. The PI3K/Akt, for its part, does not appear to partake in the hCG-induced upregulation of PGT transcripts in MGCs.

### EXAMPLE 5 - Intracellular signaling pathways that regulate PGT expression

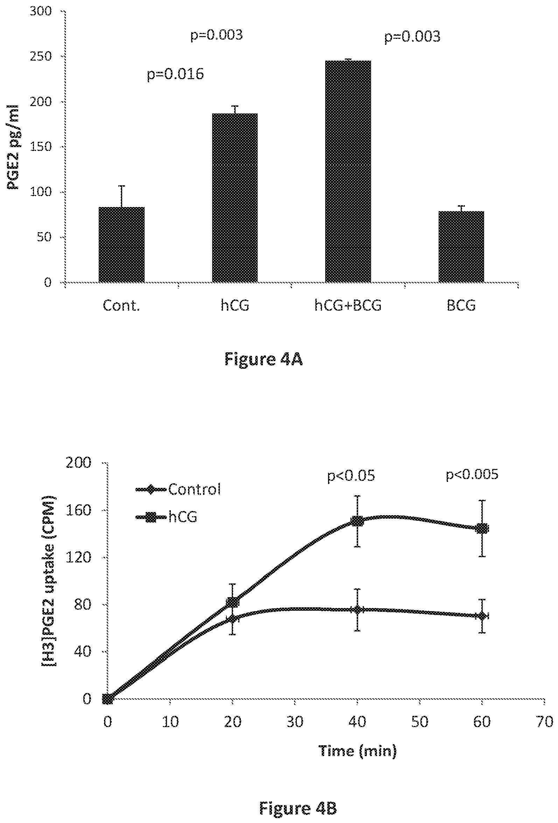

To investigate whether PGT mediates the efflux or influx of PGE₂, FSH-pretreated MGCs (48 hours) were re-incubated with hCG (to induce PGT expression; 1U/ml) for 24 hours in the absence or presence of the PGT inhibitor bromocresol green (BCG; 50mM). The concentration of PGE₂ in the media was then measured by an enzyme immunoassay (EIA). As shown in Fig. 4A, treatment with hCG produced a 2-fold increase in the concentration of PGE₂ in conditioned media. Co-treatment with BCG resulted in an additional significant (p<0.01) increase in the media concentration of PGE₂ (by ~3-fold relative to controls). In contrast, treatment with BCG by itself was without effect on the basal media concentration of PGE₂. These observations suggest that the inhibition of PGT function increases the extracellular concentration of PGE₂ and that PGT mediates the uptake of PGE₂.

To confirm the role of PGT in PGE₂ uptake, complementary influx experiments were also carried out. Cultured FSH-pretreated MGCs (48 hours) were initially treated with or without hCG (1U/ml) for 24 hours (to induce PGT expression) and then re-incubated with 1.5nM of [³H]PGE₂ for 60 minutes. Intracellular [³H]PGE₂ levels were measured at intervals over the 60 minutes incubation period. As shown in Fig. 4B, rapid initial accumulation of [³H]PGE₂ was observed over the first 20 minutes of incubation in both control and hCG-treated MGCs. However, thereafter, continued [³H]PGE₂ influx was noted only in hCG-treated MGCs for which a plateau was noted at the time point of 40 minutes. In light of the ability of hCG to upregulate the expression of PGT in MGCs, these observations suggest that the higher uptake of [³H]PGE₂ by hCG-treated MGCs is likely mediated by PGT.

### EXAMPLE 6 - In vivo regulation of ovarian PGT expression

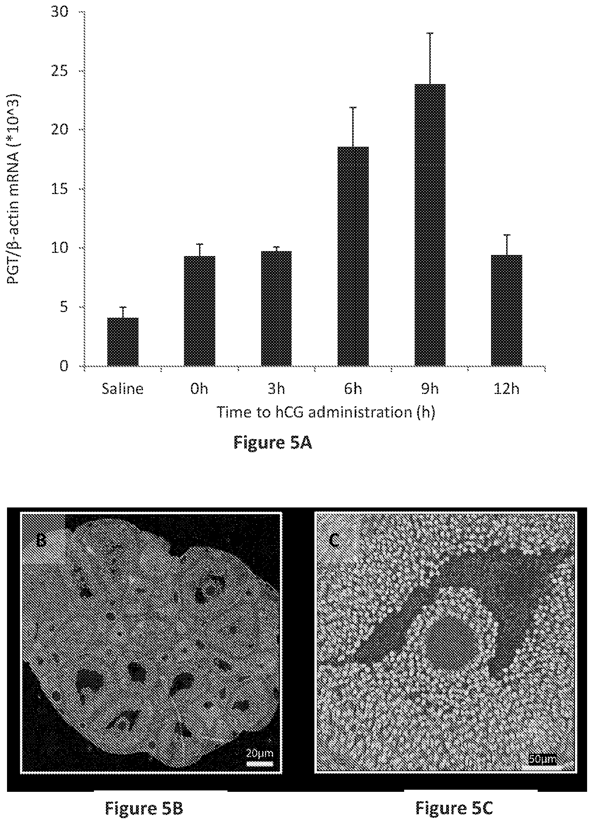

Fig. 5A presents the regulation ovarian PGT expression in vivo. Pregnant mare serum gonadotropin (PMSG)-primed/hCG-triggered was applied to immature mouse superovulation model for 48 hours (Fig. 5A, t=0) which augmented the level of PGT in the ovaries, relative to control (Fig. 5A, saline) by 2.3 folds (p<0.02). Administration of hCG further augmented the whole ovarian PGT transcripts to a peak noted 9 hours later (5.8 fold, p<0.003). Whole ovarian PGT transcripts were back at baseline levels as early as 12 hours after hCG administration.

### EXAMPLE 7 - The effect of PGT blocking on ovulation in vivo

A role for PGT in ovulation was further investigated in vivo using the PGT inhibitors 4,4'-diisothiocyanatostilbene-2,2'-disulfonic acid (DIDS) and BCG. PMSG - primed/hCG-triggered immature female mice were concurrently treated with different concentrations of DIDS or vehicle at the time of hCG administration. 16 hours later, the mice were sacrificed, ovaries were paraffin-embedded, and the number of ovulated oocytes within the ampulla of the fallopian tube was obtained. As shown in Table 2, treatment with DIDS produced dose-dependent decrements in the ovulated oocyte count peaking at the 50 mg/kg dose level (95.5% inhibition).

**Table 2- Mice ovulation upon treatment with DIDS**

| | | **No. ovulating mice/no. treated** | **No. ova/ovulating mice (Mean± SEM)** | **No. of oocytes in ovulating mice** |
|---|---|---|---|---|
| hCG | | 16/16 100% | 56.2±6.6 | 10-111 |
| hCG + Vehicle | | 5/5 (100%) | 60.6±3.5 | 55-74 |
| hCG + DIDS mg/kg) | (50 | 1/22 (4.5%) | 9 | - |
| hCG + DIDS mg/kg) | (20 | 7/12 (58.3%)^{∗} | 22 ±6.8 | 1-51 |
| hCG + DIDS mg/kg) | (10 | 9/13 (69.2%) | 27.4 ±6.8 | 10-60 |
| hCG + DIDS mg/kg) | (5 | 12/12 (100%) | 53.5 ±5.1 | 7-77 |

| | | | | |
|---|---|---|---|---|
| ^{∗}Significantly different from controls (P < 0.05) | | | | |

Moreover, the cumulus-oocyte-complexes recovered from mice treated with lower doses of DIDS displayed expanded normal-appearing cumuli. The denuded oocytes from these cumulus-oocyte-complexes were deemed morphologically normal, mature (M2), and comparable to those derived from hCG-treated mice.

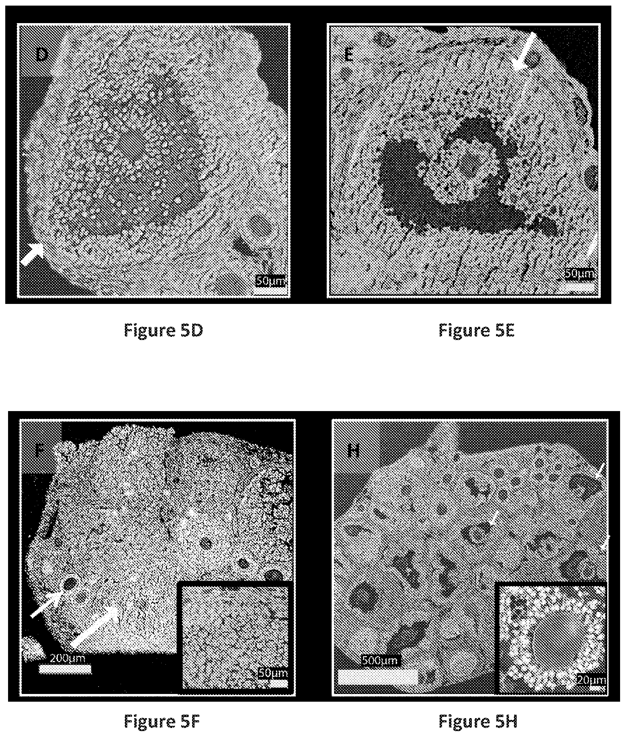

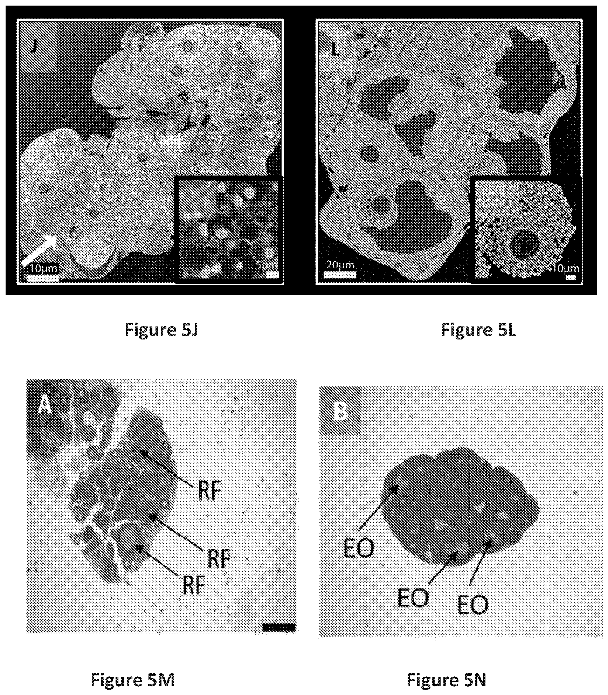

To further validate the ovulation-blocking activity of DIDS, ovarian morphology was evaluated. Treatment with PMSG yielded numerous preovulatory follicles with their follicular wall composed of a thick granulosa cells layer and an oocyte that was surrounded with compact cumulus cells (Figs. 5B and 5C). Twelve hours after the administration of hCG, control ovaries were still composed of numerous preovulatory follicles exhibiting a thin apical wall with only a few layers of MGCs (arrow at Fig. 5D) and a zone of tissue disruption replete with MGCs dispersed within the antral cavity, all characteristics of a follicle just prior ovulation. The CCs in turn proved highly dispersed (Fig. 5D). In contrast, the ovaries of hCG+DIDS-treated mice were noted to comprise unruptured preovulatory follicles replete with intact MGCs and thick multi-layered follicular walls (arrow at Fig. 5E), with no signs of impending ovulation (Fig. 5E). Control ovaries secured 16 hours after the administration of hCG (Fig. 5F) were composed of numerous ruptured follicles replete with early corpora lutea (thick arrows and enlarged inset at Fig. 5F). Secondary follicles were still present thereby indicating that the otherwise tertiary follicles have ruptured (thick arrows at Fig. 5F). In contrast, the ovaries of hCG+DIDS-treated mice (Fig. 5H) comprised numerous unruptured preovulatory follicles with entrapped oocytes (arrows and enlarged inset at Fig. 5H) replete with entrapped oocytes. The small follicles remained unaffected. The oocytes remained in the GV stage replete with a compact cumulus. Forty hours after hCG injection, control ovaries from hCG-treated mice revealed many corpora lutea comprised of luteinized granulosa cells. The corpora lutea were composed of cells containing lipid droplets in the cytoplasm (Fig. 5J). In contrast, ovaries from hCG+DIDS-treated mice still contained entrapped oocytes in preovulatory follicles (Fig. 5L).

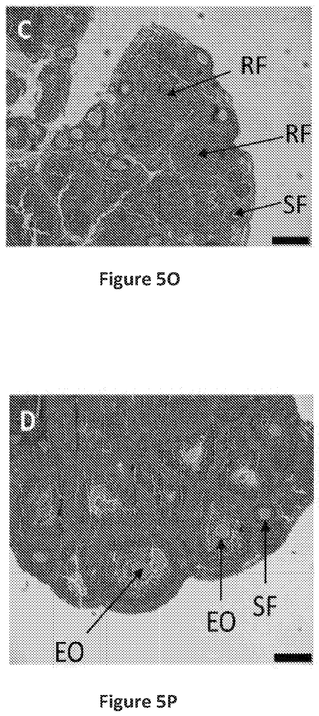

Histological examination of the effect of BCG co-injection on superovulated mice is demonstrated in Figs. 5M-5P). Immature mice were primed with 10 U of PMSG, followed by the administration of 10 U of hCG with or without the PGT blocker BCG (350 mg/kg). The ovarian photomicrographs reflect a representative experiment out of a total of at least three independent experiments. Figure 5M is a photomicrograph taken 16 hours after the administration of hCG, where representative raptured follicles can be seen (RF), next to several small secondary follicles (scale bar = 500 µm). Figure 5N is a photomicrograph taken 16 hours after the administration of hCG+BCG, showing the presence of un-ruptured follicles with entrapped oocytes (EO; scale bar = 500 µm). Figures 5O and 5P are high magnification photomicrographs of postovulatory follicle 16 hours after the administration of hCG and hCG+BCG, respectively (EO - Entrapped oocyte; RF - ruptured follicle; SF - secondary follicle; scale bar = 200 µm).

The ovulation-blocking activity of DIDS was further evaluated by examining its effect on oocyte maturation and cumulus expansion. Ovulated COCs from hCG-treated mice were collected from the ampulla of the oviduct. As expected, all COCs from hCG-treated mice displayed an expanded cumulus. In contrast, all COCs from mice treated with hCG and DIDS (50 mg/kg) displayed a compact cumulus (Table 3). Oocytes from the hCG-treated group were mature and beyond the GV breakdown stage (Table 3) while all of the oocytes from the hCG+DIDS-treated mice proved to be immature and at the GV stage (Table 3). Likewise, all of the DIDS-treated COCs examined featured a compact cumulus even at 24 hours following treatment with hCG in contrast to the expanded COCs obtained from hCG-treated mice not receiving DIDS (Table 3).

**Table 3- DIDS inhibits mice oocyte maturation in vivo**

| | GV | MI | MII |
|---|---|---|---|
| hCG | 0% (0/93) | 38% (36/93) | 62% (57/93) |
| hCG+DIDS | 100% (102/102) | 0% (0/102) | 0% (0/102) |

To further validate the ovulation-blocking effect of PGT inhibitors, mice were treated with hCG, and further with or without BCG. The mice were sacrificed 16 hours later and the number of ovulated oocytes within the tubal ampulla counted. As shown in Table 4, treatment with BCG at concentrations of 500 and 350mg/kg resulted in the complete blockade of ovulation. Paraffin-embedded sections of ovaries from BCG-treated mice revealed entrapped follicles as previously noted in ovaries of DIDS-treated counterparts (Figs. 5M-5P).

**Table 4- BCG inhibits mice ovulation**

| | | No. ovulating mice/no. treated | No. ova/ovulating mice (Mean ± SEM) | Range of oocytes in ovulating mice |
|---|---|---|---|---|
| hCG | | 15/15 (100%) | 55.9±6.6 | 10-111 |
| hCG + BCG mg/kg) | (500 | 0/4 (0%)^{∗} | 0^{∗} | - |
| hCG + BCG mg/kg) | (350 | 0/4 (0%)^{∗} | 0^{∗} | - |
| hCG + BCG mg/kg) | (300 | 3/10 (30%)^{∗} | 1.3^{∗} | 1-2 |
| hCG + BCG mg/kg) | (250 | 15/16 (93.8%) | 58.4 ±7.4 | 12-61 |

| | | | | |
|---|---|---|---|---|
| Significantly different from controls (P < 0.05) | | | | |

### EXAMPLE 8 - The effect of DIDS on PGE₂ downstream target genes

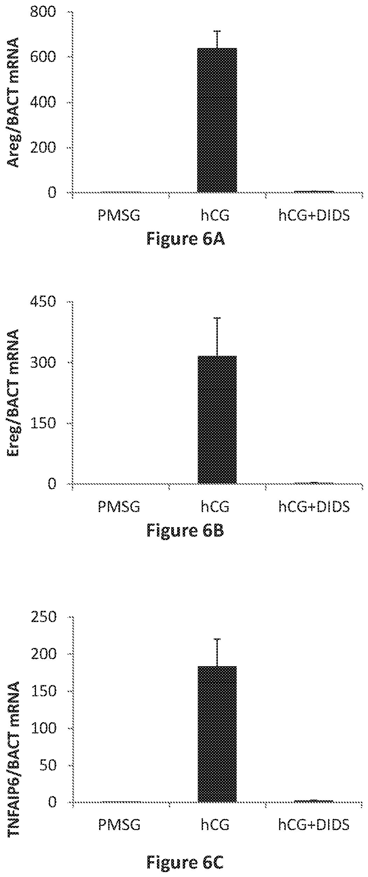

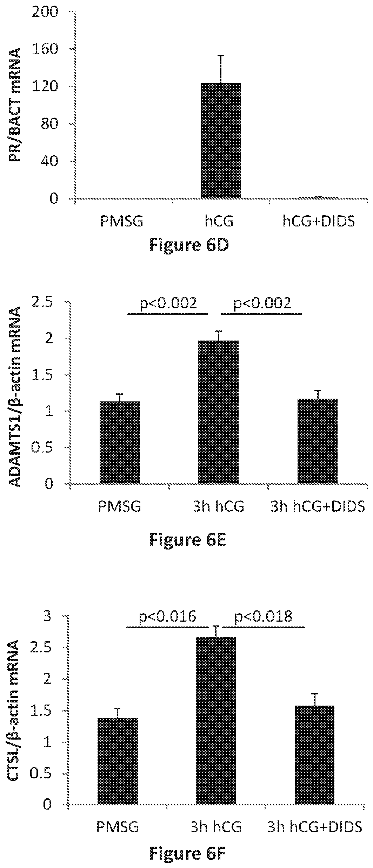

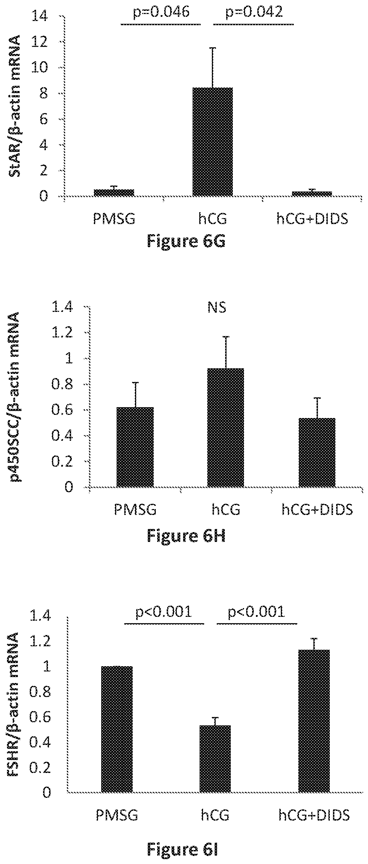

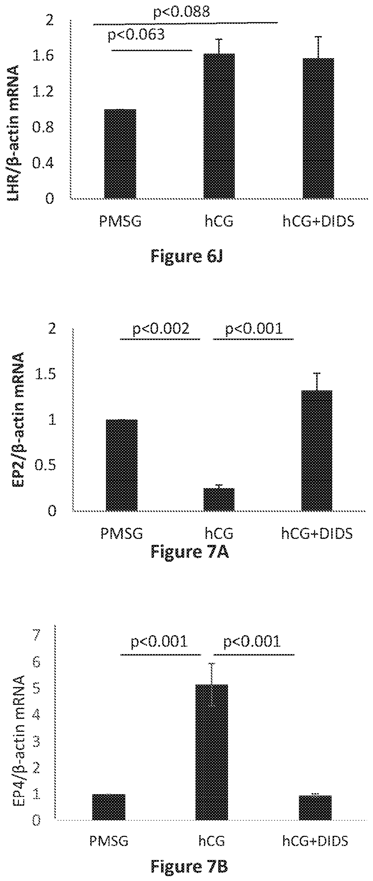

To characterize the ovulation-blocking effect of PGT at the molecular level, the effect of DIDS on the LH/CGR dependent activities was evaluated. Key representative genes of the ovulatory cascade were studied, including, oocyte maturation genes: amphiregulin (AREG), epiregulin (EREG), progesterone receptor (PR); cumulus expansion genes: tumor necrosis factor alpha-induced protein (TNFAIP6); follicular rupture related genes: disintegrin and metalloproteinase with thrombospondin motifs 1 (ADAMTS1), cathepsin L (CTSL); the ovulatory related genes: follicle stimulating hormone receptor (FSHR) and luteinizing hormone/chorionic gonadotropin receptor (LHCGR); and steroidogenesis related genes: steroidogenic acute regulatory protein (StAR) and cholesterol side cleavage chain (p450SCC). After total RNA was extracted from the ovaries of PMSG-primed/hCG+DIDS-treated mice and converted to cDNA, the expression of genes of interest was quantified by qPCR. As shown in Figs. 6A-6J, treatment with the PGT inhibitor DIDS has given rise to profound inhibition of the expression of most of the ovulation related transcripts studied with the exception of p450SCC and LHCGR which were not upregulated by hCG either. The reduction of FSHR mRNA in response to hCG treatment was abolished by the addition of DIDS.

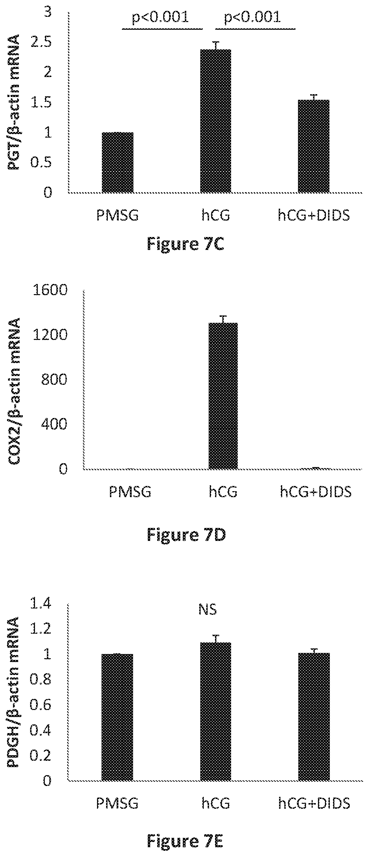

Analysis of PG receptors and PG metabolism genes (Figs. 7A-7E) revealed that the downregulation of EP2 by hCG was abolished by the addition of DIDS, that the upregulation by hCG of EP4, COX-2 and PGT was inhibited by DIDS, and that PGDH expression was not affected by either hCG or DIDS.

### EXAMPLE 9 - The effect of PGT inhibitor DIDS on corpus luteum function

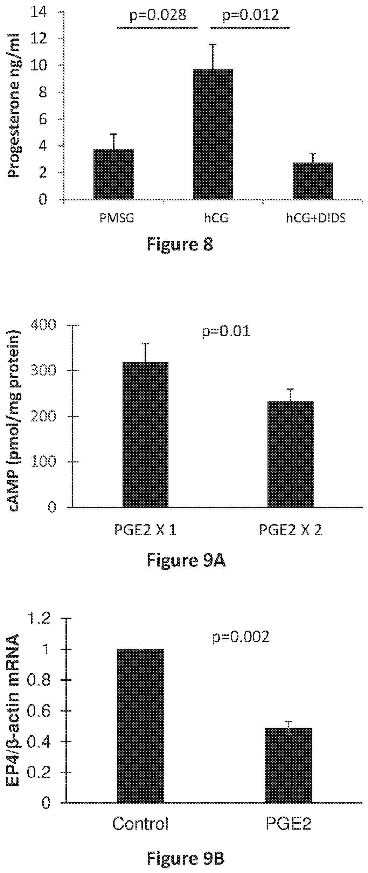

In order to assess corpus luteum function, serum progesterone levels were measured in PMSG-primed/hCG-triggered mice in the absence or presence of DIDS. As shown in Fig. 8, serum progesterone levels were significantly increased 24 hours post-hCG injection (p=0.03). However, concurrent treatment with DIDS resulted in significant reduction in serum progesterone levels (p=0.01).

### EXAMPLE 10 - The effect of PG receptor desensitization in human granulosa cells

Inhibition of PGT activity or the provision of excess PGE₂ results in the desensitization of the prostaglandin receptors EP1 and EP4 and thereby in a reduction in PGE₂ signaling. An important second messenger for PGE₂ is intracellular cAMP resulting from activation of either EP2 or EP4 receptor by PGE₂. Thus, the hypothesis that excess PGE₂ results in reduced PG signaling in human granulosa cells was tested. MGCs were incubated with 10 µM PGE₂ for 10 min or pretreated with 10 µM PGE₂ for 10 min, washed and then treated with 10 µM PGE₂ for additional 10 min. cAMP levels were measured in the cell lysate and was normalized to total protein content. As shown in Fig. 9A, pretreatment of cells with PGE2 for 10 min leads to significantly reduced accumulation of intracellular cAMP in response to further PGE₂ stimulation, demonstrating EP receptor desensitization (p=0.01).

Long-term desensitization by excess PGE₂ on EP4 receptor expression in human granulosa cells was also examined. FSH-pretreated MGCs (48 hours) were re-incubated with 10 µM PGE₂ for additional 24 hours. As shown in Fig. 9B, treatment of MGCs with PGE₂ resulted in significant downregulation of EP4 receptor mRNA expression compared to controls (p=0.0002).

## Claims

1. Use of a pharmaceutical composition as a contraceptive in a female subject comprising at least one PGT inhibitor and a pharmaceutically acceptable carrier, excipient or diluent.

2. The use of claim 1, wherein said PGT inhibitor is capable of inhibiting at least one of: oocyte nuclear maturation, cumulus expansion, and follicular rupture.

3. The use of any one of claims 1 to 2, wherein said PGT inhibitor is capable of inhibiting the expression of one or more of steroidogenesis related genes, oocyte maturation genes, cumulus expansion genes and follicular rupture genes.

4. The use of any one of claims 1 to 3, wherein said PGT inhibitor is capable of inhibiting ovulation, and wherein said ovulation is a spontaneous ovulation.

5. The use of claim 4, wherein said female subject is undergoing fertility treatments.

6. The use of any one of claims 1 to 5, wherein said pharmaceutical composition is for daily use during one to four days prior the mid-cycle.

7. The use of any one of claims 1 to 6, wherein said pharmaceutical composition is for daily use during mid-cycle and at least one day thereafter.

8. The use of any one of claims 1 to 5, for daily use from day 9 of the menstrual cycle to day 14 of the menstrual cycle.

9. The use of any one of claims 1 to 5, for daily use from the day that the level of LH surges until, and including, the day that LH level drops to baseline.

10. The use of any one of claims 1 to 5, for daily use, at the day that the level of estradiol surges and at least four consecutive days thereafter.

11. The use of any one of claims 1 to 5, for daily use at the day that the level of estradiol surges and until the day that the level of LH drops to baseline.

12. The use of any one of claims 1 to 11, wherein the at least one PCT inhibitor is selected from the group consisting of an anion exchange inhibitor and a triarylmethane dye.

13. The use of claim 12, wherein said anion exchange inhibitor is 4,4'-diisothiocyanatostilbene-2,2'-disulfonic acid (DIDS).

14. The use of claim 12, wherein said triarylmethane dye is bromocresol green (BCG).

15. A pharmaceutical composition comprising at least one PGT inhibitor and a pharmaceutically acceptable carrier, excipient or diluent, for use in suppressing spontaneous ovulation during fertility treatments.

## Patentansprüche

1. Verwendung einer pharmazeutischen Zusammensetzung als einem Kontrazeptivum bei einem weiblichen Subjekt, umfassend mindestens einen PGT-Inhibitor und einen pharmazeutisch annehmbaren Träger, Hilfsstoff oder Verdünnungsmittel.

2. Verwendung nach Anspruch 1, wobei der PGT-Inhibitor zur Hemmung mindestens eines von Kernreifung von Eizellen, Cumulusexpansion und Follikelsprung fähig ist.

3. Verwendung nach einem der Ansprüche 1 bis 2, wobei der PGT-Inhibitor zur Hemmung der Expression eines oder mehrerer von für die Steroidgenese relevanten Genen, Eizellreifungsgenen, Cumulusexpansionsgenen und Follikelsprunggenen fähig ist.

4. Verwendung nach einem der Ansprüche 1 bis 3, wobei der PGT-Inhibitor zur Hemmung des Eisprungs fähig ist, und wobei der Eisprung ein spontaner Eisprung ist.

5. Verwendung nach Anspruch 4, wobei das weibliche Subjekt derzeit Fruchtbarkeitsbehandlungen unterzogen wird.

6. Verwendung nach einem der Ansprüche 1 bis 5, wobei die pharmazeutische Zusammensetzung für die tägliche Anwendung während ein bis vier Tagen vor der Zyklusmitte ist.

7. Verwendung nach einem der Ansprüche 1 bis 6, wobei die pharmazeutische Zusammensetzung für die tägliche Anwendung während der Zyklusmitte sowie mindestens einen Tag danach ist.

8. Verwendung nach einem der Ansprüche 1 bis 5, für die tägliche Anwendung von Tag 9 des Menstruationszyklus bis Tag 14 des Menstruationszyklus.

9. Verwendung nach einem der Ansprüche 1 bis 5, für die tägliche Anwendung von dem Tag, an dem der LH-Spiegel ansteigt, bis, und einschließlich, zu dem Tag, an dem der LH-Spiegel auf die Grundlinie abfällt.

10. Verwendung nach einem der Ansprüche 1 bis 5, für die tägliche Anwendung an dem Tag, an dem der Estradiolspiegel ansteigt, sowie mindestens vier aufeinander folgende Tage danach.

11. Verwendung nach einem der Ansprüche 1 bis 5, für die tägliche Anwendung an dem Tag, an dem der Estradiolspiegel ansteigt, und bis zu dem Tag, an dem der LH-Spiegel auf die Grundlinie abfällt.

12. Verwendung nach einem der Ansprüche 1 bis 11, wobei der mindestens eine PCT-Inhibitor ausgewählt ist aus der Gruppe bestehend aus einem Anionenaustausch-Inhibitor und einem Triarylmethan-Farbstoff

13. Verwendung nach Anspruch 12, wobei der Anionenaustausch-Inhibitor 4,4'-Diisothiocyanatostilben-2,2'-disulfonsäure (DIDS) ist.

14. Verwendung nach Anspruch 12, wobei der Triarylmethan-Farbstoff Bromcresolgrün (BCG) ist.

15. Pharmazeutische Zusammensetzung, umfassend mindestens einen PGT-Inhibitor und einen pharmazeutisch annehmbaren Träger, Hilfsstoff oder Verdünnungsmittel, zur Verwendung bei der Unterdrückung eines spontanen Eisprungs während Fruchtbarkeitsbehandlungen.

## Revendications

1. Utilisation d'une composition pharmaceutique en tant que contraceptif chez un sujet féminin comprenant au moins un inhibiteur des PGT et un vecteur, un excipient ou un diluant pharmaceutiquement acceptables.

2. Utilisation selon la revendication 1, dans laquelle ledit inhibiteur des PGT est capable d'inhiber au moins l'une de : la maturation nucléaire des ovocytes, l'expansion du cumulus, et la rupture folliculaire.

3. Utilisation selon l'une quelconque des revendications 1 à 2, dans laquelle ledit inhibiteur des PGT est capable d'inhiber l'expression d'un ou plusieurs des gènes associés à la stéroïdogenèse, des gènes de maturation des ovocytes, des gènes d'expansion du cumulus et des gènes de rupture folliculaire.

4. Utilisation selon l'une quelconque des revendications 1 à 3, dans laquelle ledit inhibiteur des PGT est capable d'inhiber l'ovulation, dans laquelle ladite ovulation est une ovulation spontanée.

5. Utilisation selon la revendication 4, dans laquelle ledit sujet féminin subit des traitements pour la fertilité.

6. Utilisation selon l'une quelconque des revendications 1 à 5, dans laquelle ladite composition pharmaceutique est destinée à une utilisation quotidienne pendant un à quatre jours avant le milieu du cycle.

7. Utilisation selon l'une quelconque des revendications 1 à 6, dans laquelle ladite composition pharmaceutique est destinée à une utilisation quotidienne au milieu du cycle et au moins un jour après cela.

8. Utilisation selon l'une quelconque des revendications 1 à 5, pour une utilisation quotidienne à partir du jour 9 du cycle menstruel jusqu'au jour 14 du cycle menstruel.

9. Utilisation selon l'une quelconque des revendications 1 à 5, pour une utilisation quotidienne à partir du jour d'augmentation du niveau de LH, et y compris le jour auquel le niveau de LH retourne à sa valeur de base.

10. Utilisation selon l'une quelconque des revendications 1 à 5, pour une utilisation quotidienne, le jour où le niveau d'estradiol augmente et au moins pendant 4 jours consécutifs à partir de là.

11. Utilisation selon l'une quelconque des revendications 1 à 5, pour une utilisation quotidienne le jour où le niveau d'estradiol augmente jusqu'au jour où le niveau de LH retourne à sa valeur de base.

12. Utilisation selon l'une quelconque des revendications 1 à 11, dans laquelle l'au moins un inhibiteur des PGT est sélectionné dans le groupe consistant en un inhibiteur d'échange d'anions et un colorant triarylméthane.

13. Utilisation selon la revendication 12, dans laquelle ledit inhibiteur d'échange d'anions est l'acide 4,4'-diisothiocyanatostilbène-2,2'-disulfonique (DIDS).

14. Utilisation selon la revendication 12, dans laquelle ledit colorant triarylméthane est le vert de bromocrésol (BCG).

15. Composition pharmaceutique comprenant au moins un inhibiteur des PGT et un vecteur, un excipient ou un diluant pharmaceutiquement acceptables, pour son utilisation dans la suppression de l'ovulation spontanée pendant des traitements pour la fertilité.
